# EUROPEAN PATENT APPLICATION

(11) **EP 4 130 238 A1**
(43) Date of publication of application: **08.02.2023**
(21) Application number: 21775909.1
(22) Date of filing: 24.03.2021
(51) Int. Cl.: C12N 5/00, C12N 5/0735

(54) **HEPES-CONTAINING MEDIUM**

(30) Priority: 25.03.2020 JP 2020054572
(71) Applicant: AJINOMOTO CO., INC., Chuo-ku Tokyo 104-8315 (JP)
(72) Inventor: SUZUKI, Yu, Kawasaki-shi, Kanagawa 210-8681 (JP); KITAZAWA, Manabu, Kawasaki-shi, Kanagawa 210-8681 (JP); OGAWA, Shimpei, Kawasaki-shi, Kanagawa 210-8681 (JP)
(74) Representative: Hoffmann Eitle
(86) International application number: PCT/JP2021/012327
(87) International publication number: WO 2021/193748

(57) **Abstract**

The present invention aims to provide a medium for cell culture that enables cell culture without exhibiting toxicity when HEPES buffer is used at a high concentration, a production method of the medium, and a cell culture method using the medium. A cell proliferation rate can be improved by a medium for cell culture, containing 4-(2-hydroxyethyl)-1-piperazineethanesulfonic acid (HEPES) at a concentration of not less than 30 mM, and having an osmotic pressure of 220 to 340 mOsm/kg, and a cell culture method using the medium.

## Description

### [Technical Field]

The present invention relates to a medium for cell culture, particularly a medium for cell culture containing HEPES, and a production method of the medium.

### [Background Art]

Cell culture means proliferating and maintaining cells isolated from living tissue in a culture medium, and it is important to select a culture environment, a culture medium, and a base material that are appropriate for individual cells and establish a survival environment.

Survival environments are broadly classified into physicochemical environments (e.g., temperature, pH, osmotic pressure, oxygen partial pressure and carbon dioxide partial pressure) and physiological environments (e.g., concentrations of hormones and nutrients), and these environments (other than temperature) can be generally controlled by the composition of the medium.

Most of the normal mammalian cell lines grow well at about pH 7.4 (pH 7.3 to 7.5). Generally, this buffering action is achieved by including a buffering agent/buffer in the medium.

As the buffering agents/buffers to be contained in the media for cell culture, various ones have been used, and carbon dioxide-bicarbonate-based buffers, phosphate buffers, acetic acid-sodium acetate-based buffers, HEPES (4-(2-hydroxyethyl)-1-piperazineethanesulfonic acid) buffers, citrate buffers, Tris-HCl buffers, and the like are used alone or in combination.

In carbon dioxide-bicarbonate-based buffers, since pH is adjusted under a 5% CO₂ atmosphere (conventional carbon dioxide concentration in cell culture), it easily changes as the cells are moved into and out from the incubator.

On the other hand, HEPES buffers can maintain a constant pH of 6.8 to 8.2 regardless of the gas partial pressure. Due to such superior buffering action, HEPES buffers are widely used in a supplementary role to other buffers such as sodium hydrogen carbonate. However, the toxicity thereof (Non Patent Literatures 1 to 5) is feared, and many are used at HEPES concentrations in the range of 10 to 25 mM (e.g., DMEM_Lonza 12-708F, DMEM/F-12_Lomza 12-719F, mTeSR STEMCELL Technologies ST-85850).

In recent years, in the search for high density culture methods aiming at practicalization of regenerative medicine, it has become clear that the pH buffering action of conventional buffer formulations is highly likely insufficient. In addition, the number of cases where culture in a medium with high buffer potency is required, such as iPS cells, is increasing.

### [Citation List]

### [Non Patent Literature]

[NPL 1]
   Poole CA, et al., In Vitro. 1982 Sep; 18(9):755-65.
[NPL 2]
   Zigler JS Jr, et al., In Vitro Cell Dev Biol. 1985 May; 21 (5) :282-7.
[NPL 3]
   Bowman CM, et al., In Vitro Cell Dev Biol. 1985 Mar; 21(3 Pt 1) :140-2.
[NPL 4]
   Hanrahan JW, et al., J Membr Biol. 1990 Jun; 116(1):65-77.
[NPL 5]
   R Depping, Analytical and Bioanalytical Chemistry, 2019, Volume 411, Issue 4, pp 797-802

### [Summary of Invention]

### [Technical Problem]

The present invention aims to provide a medium for cell culture that enables cell culture without exhibiting toxicity when HEPES buffer is used at a concentration not less than that conventionally used in expectation of an enhanced buffering action. Furthermore, the present invention aims to provide a production method of such a medium for cell culture, and a cell culture method using the medium.

### [Solution to Problem]

The present inventors in expectation of an enhanced buffering action first tried a cell culture at a HEPES concentration higher than the concentration recommended for cell culture. As a result, the cell growth deteriorated as feared. On the other hand, it was found that when the cells were cultured at a high HEPES concentration, the cells grew poorly and the osmotic pressure of the medium exceeded the optimum range. Based on these findings, the present inventors have conducted intensive studies in view of the above-mentioned problems and found that the expression of toxicity can be suppressed even when cultured in a high HEPES concentration environment, by adjusting the osmotic pressure to fall within an optimal range, which resulted in the completion of the present invention.

Accordingly, the present invention provides the following.
[1] A medium for cell culture, comprising 4-(2-hydroxyethyl)-1-piperazineethanesulfonic acid (HEPES) at a concentration of not less than 30 mM, and having an osmotic pressure of not more than 340 mOsm/kg.
[2] The medium of the above-mentioned [1], wherein the osmotic pressure is not less than 220 mOsm/kg.
[3] The medium of the above-mentioned [1] or [2], wherein the HEPES concentration is not more than 190 mM.
[4] The medium of any of the above-mentioned [1] to [3], wherein the HEPES concentration is not less than 30 mM and not more than 190 mM.
[5] The medium of any of the above-mentioned [1] to [4], wherein the cell is a pluripotent stem cell.
[6] The medium of the above-mentioned [5], wherein the pluripotent stem cell is an iPS cell.
[7] A method for producing a medium for cell culture, comprising adding HEPES to a basal medium at a final concentration of not less than 30 mM, and further adjusting the osmotic pressure to not more than 340 mOsm/kg.
[8] The method of the above-mentioned [7], wherein the osmotic pressure is not less than 220 mOsm/kg.
[9] The method of the above-mentioned [8], wherein the osmotic pressure is adjusted by adjusting a concentration of sodium chloride in the medium.
[10] The method of any of the above-mentioned [7] to [9], wherein the HEPES concentration is not more than 190 mM.
[11] The method of any of the above-mentioned [7] to [10], wherein the HEPES concentration is not less than 30 mM and not more than 190 mM.
[12] The method of any of the above-mentioned [7] to [11], wherein the cell is a pluripotent stem cell.
[13] The method of the above-mentioned [12], wherein the pluripotent stem cell is an iPS cell.
[14] A method for culturing a cell, comprising culturing in a medium of any of the above-mentioned [1] to [4].
[15] The method of the above-mentioned [14], wherein the cell is a pluripotent stem cell.
[16] The method of the above-mentioned [15], wherein the pluripotent stem cell is an iPS cell.
[17] A cell-containing composition, comprising a medium of any of the above-mentioned [1] to [4] and a cell.
[18] The composition of the above-mentioned [17], wherein the cell is a pluripotent stem cell.
[19] The composition of the above-mentioned [18], wherein the pluripotent stem cell is an iPS cell.
[20] A method for preparing a cell, comprising a step of culturing and proliferating the cell in a medium of any of the above-mentioned [1] to [4].
[21] The method of the above-mentioned [20], wherein the cell is a pluripotent stem cell.
[22] The method of the above-mentioned [21], wherein the pluripotent stem cell is an iPS cell.

### [Advantageous Effects of Invention]

According to the present invention, it is possible to provide a medium with improved pH stability due to a superior buffering action afforded by a high HEPES concentration. Furthermore, the medium for cell culture, the cell culture method, and the like of the present invention can improve the cell proliferation rate. Therefore, a larger number of cells, particularly pluripotent stem cells such as iPS cells and the like, can be obtained, and a large amount of the cells can be supplied for use in research, medical care, and the like.

### [Brief Description of Drawings]

[Fig. 1]

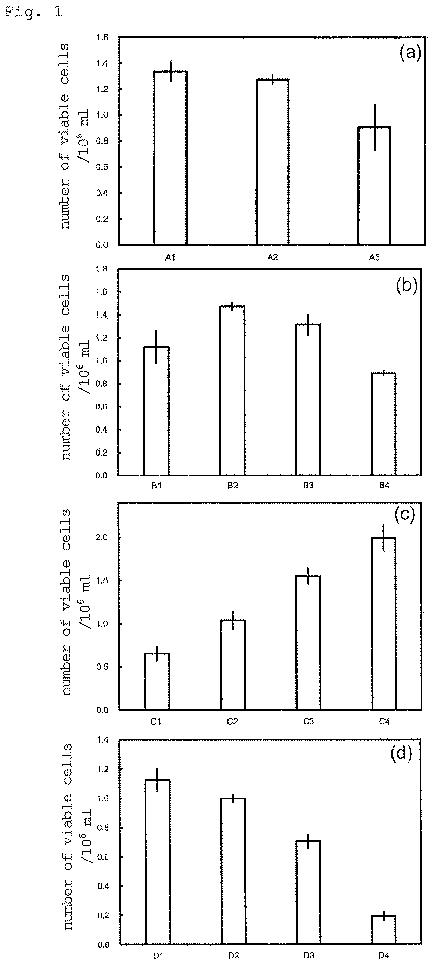

   Fig. 1 is a graph showing the number of viable cells obtained by detaching all the cells in one well, suspending them in 1 ml of medium, and counting them. count (n=3) at the time point of d6 in culture using (a) media A1 to A3 (VTN-N), and count (n=3) at the time point of d7 in culture using (b) media B1 to B4 (iMatrix), (c) media C1 to C4 (VTN-N), and (d) media C1 to C4 (iMatrix). The vertical axis shows the number of viable cells, and the horizontal axis shows the kind of medium. VTN-N shows use of an rh-VTN-N-coated culture container, and iMatrix shows use of an iMatrix-511-coated culture container.
[Fig. 2]

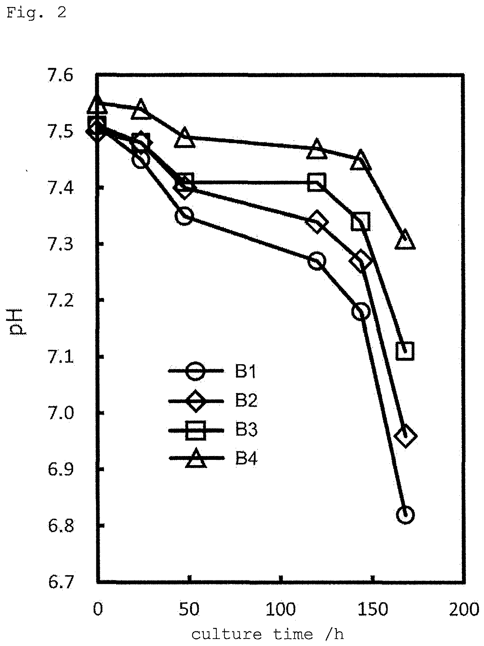

   Fig. 2 is a graph showing the pH value of the culture supernatant immediately before medium replacement. The results of culture using the media B1 to B4 are shown. The vertical axis shows pH value, and the horizontal axis shows culture period (h).
[Fig. 3]

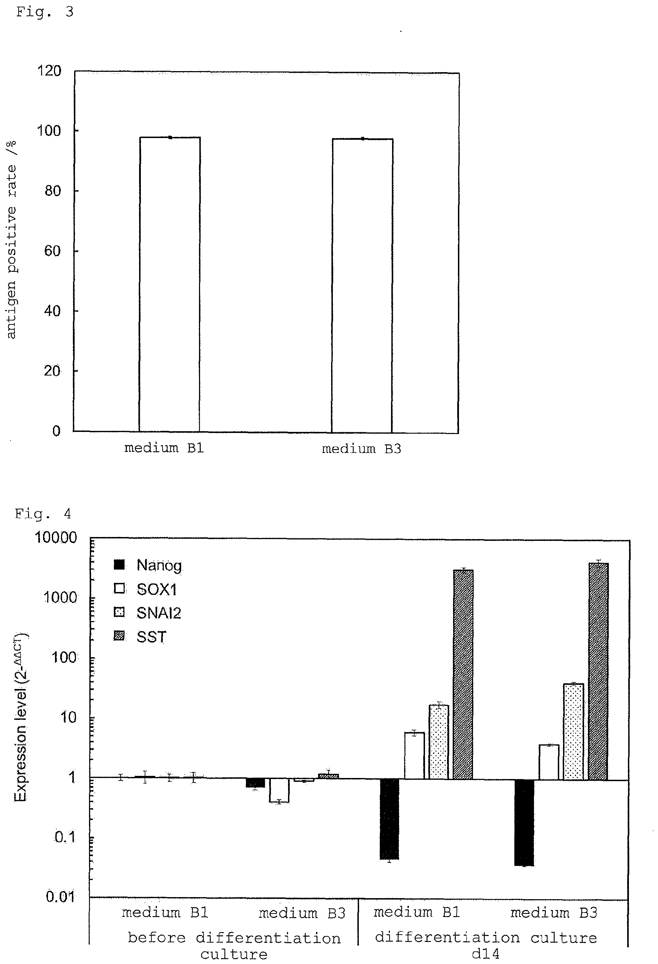

   Fig. 3 is a graph showing the CD30 antigen positive rate at the time point of d7 in human iPS cell culture using the medium B1 or B3 (n=3).
[Fig. 4]
   Fig. 4 is a graph showing the examination results of the expression levels of undifferentiation marker and differentiation markers in cells obtained by culturing human iPS cells in medium B1 or B3 for 3 weeks, switching the medium to a differentiation medium, and culturing the cells for additional 2 weeks (n=3). The relative expression levels are shown based on the state, before differentiation culture, of the cells cultured in medium B1. undifferentiation marker: Nanog, differentiation markers: SOX1, SNAI2, SST.
[Fig. 5]

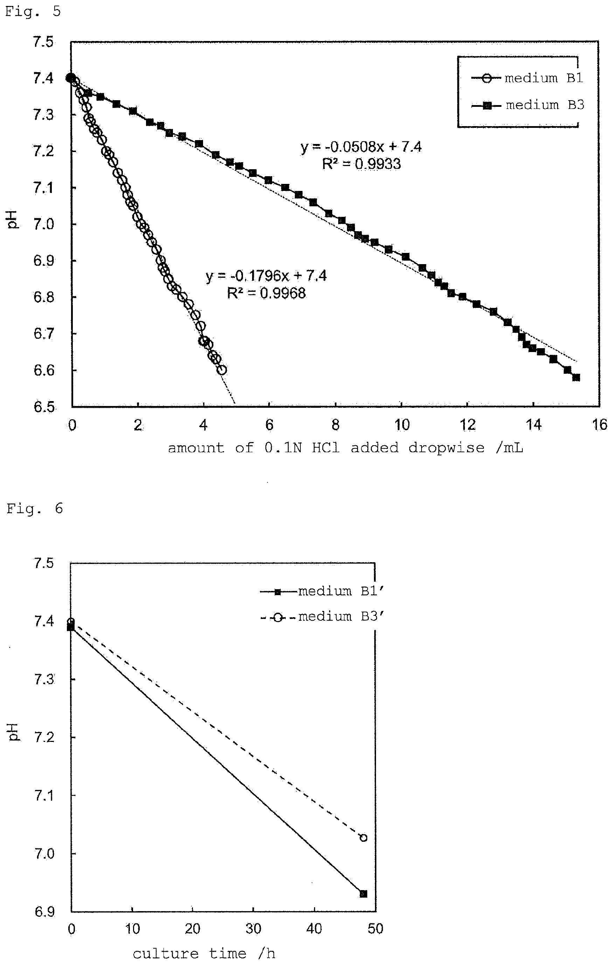

   Fig. 5 is a graph showing the measurement results of the pH buffer capacity of the medium B1 and medium B3.
[Fig. 6]
   Fig. 6 is a graph showing the pH value of the culture supernatant immediately before medium replacement. The results of suspension culture using medium B1' or B3' are shown. The vertical axis shows pH value, and the horizontal axis shows culture period (h).
[Fig. 7]

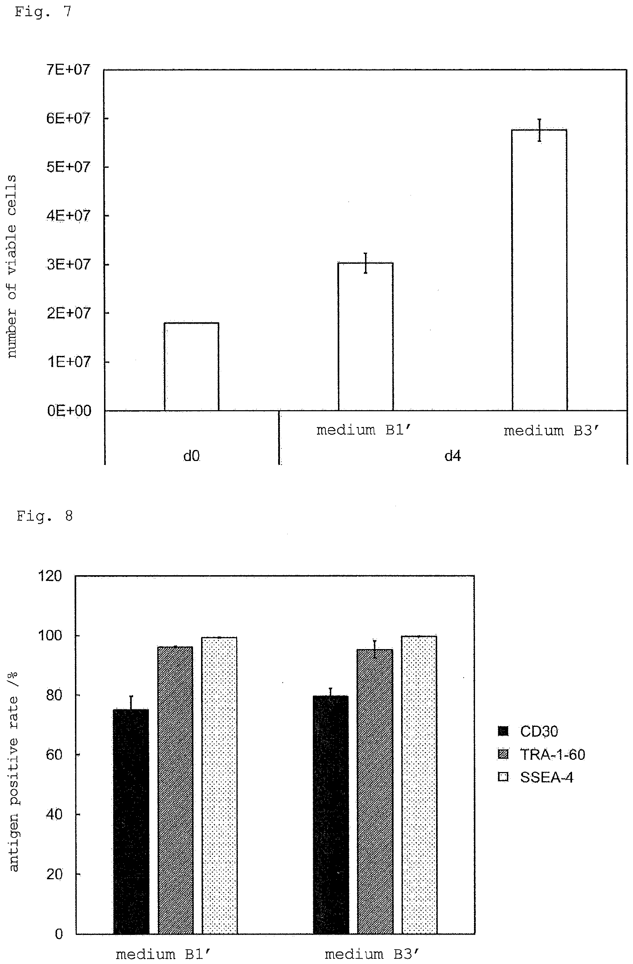

   Fig. 7 is a graph showing the number of viable cells counted by centrifuging the cell suspension after suspension culture, removing the culture supernatant, converting the cells to single cells by Accumax treatment, resuspending them in a medium, and counting. The counts (n=3) at the time point of d4 in the culture using medium B1' or B3' are shown. The vertical axis shows the number of viable cells, and the horizontal axis shows the kind of medium.
[Fig. 8]
   Fig. 8 is a graph showing the results of the expression level of undifferentiation markers examined at the time point of d4 in suspension culture of human iPS cells using medium B1' or B3' (n=3). antigens: CD30 antigen, TRA-1-60 antigen, SSEA-4 antigen.
[Fig. 9]

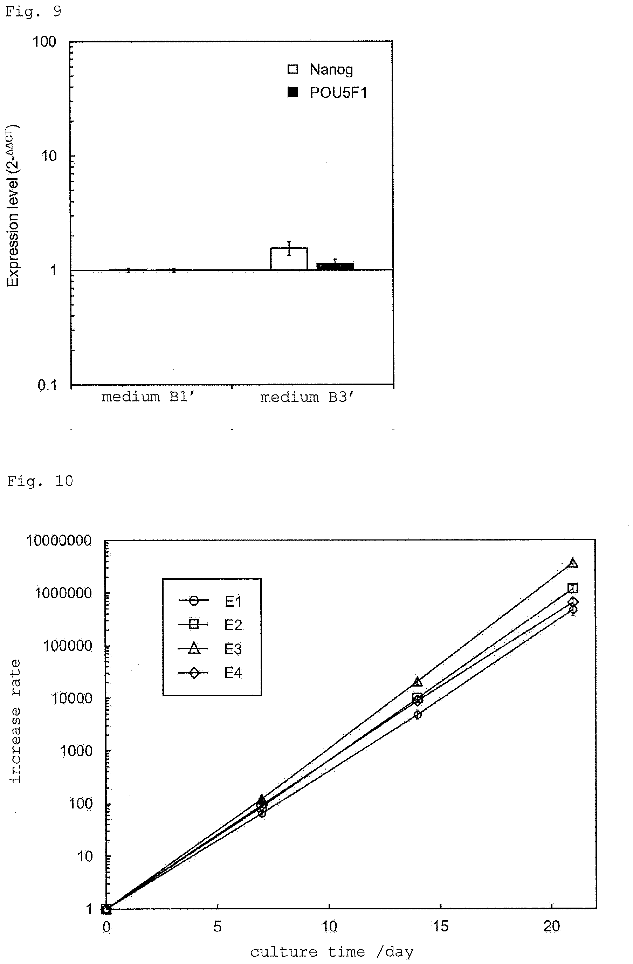

   Fig. 9 is a graph showing each antigen (surface marker) positive rate at the time point of d4 in suspension culture of human iPS cells using medium B1' or B3' (n=3). undifferentiation markers: Nanog, POU5F1.
[Fig. 10]
   Fig. 10 is a graph showing the measurement results of increase rate for each passage, after seeding human iPS cells using media E1 to E4 in a culture vessel coated with iMatrix-511 and performing passage.

### [Description of Embodiments]

The present invention is described below. Unless particularly indicated, the terms used in the present specification have meanings generally used in the pertinent field.

### 1. Medium for cell culture and production method thereof

The present invention provides a medium for cell culture, comprising 4-(2-hydroxyethyl)-1-piperazineethanesulfonic acid (HEPES) at a concentration of not less than 30 mM, and having an osmotic pressure of not more than 340 mOsm/kg (hereinafter sometimes referred to as "the medium of the present invention") and a production method of the medium (hereinafter sometimes referred to as "the production method of the present invention"). The production method of the present invention is specifically performed by adding HEPES to a basal medium at a final concentration of not less than 30 mM, and further adjusting the osmotic pressure to not more than 340 mOsm/kg. When the basal medium contains HEPES as a buffering agent, the concentration of HEPES in the medium of the present invention contains HEPES in the basal medium.

HEPES used in the present invention is a compound having pKa of 7.55 (20°C), and shows a buffering action at a pH within the range of about 6.8 to 8.2. When exposed to light for a long time, it shows toxicity derived from hydrogen peroxide (Non Patent Literature 2). Thus, it is desirable to avoid the development of hydrogen peroxide and store HEPES and liquids containing HEPES (e.g., medium containing HEPES) in the dark as much as possible.

In expectation of improved pH stability by a buffering action, the concentration of HEPES in the medium of the present invention is characteristically not less than 30 mM. It is also preferably not less than 35 mM, or not less than 40 mM.

From the aspect of osmotic pressure adjustment, the concentration of HEPES in the medium of the present invention is preferably not more than 190 mM. It is also preferably not more than 180 mM, not more than 170 mM, not more than 160 mM, not more than 150 mM, not more than 140 mM, not more than 130 mM, not more than 120 mM, not more than 110 mM, or not more than 100 mM.

The concentration of HEPES in the medium of the present invention is preferably not less than 30 mM and not more than 190 mM. It is also preferably not less than 35 mM and not more than 180 mM, not less than 40 mM and not more than 170 mM, not less than 40 mM and not more than 160 mM, not less than 40 mM and not more than 150 mM, not less than 40 mM and not more than 140 mM, not less than 40 mM and not more than 130 mM, not less than 40 mM and not more than 120 mM, or not less than 40 mM and not more than 100 mM. Particularly preferably, the concentration of HEPES in the medium of the present invention is not less than 40 mM and not more than 160 mM.

In the present specification, the "basal medium" refers to a medium containing a carbon source, a nitrogen source, an inorganic salt, and the like that are essential for culturing cells. The basal medium that can be used as a constituent component of the medium of the present invention is not particularly limited and may be appropriately selected according to the type of cells to be cultured. The basal medium may be prepared by a method known per se, or a commercially available product may also be used.

As a useable basal medium, those known per se can be mentioned, and it is preferably animal cell culture medium generally used in the pertinent field. Examples thereof include Dulbecco's modified Eagle medium (DMEM), Ham's Nutrient Mixture F12, DMEM/F12 medium, McCoy's 5A medium, Minimum Essential medium (MEM), Eagle's Minimum Essential medium (EMEM), alpha Modified Eagle's Minimum Essential medium (αMEM), Roswell Park Memorial Institute (RPMI) 1640 medium, Iscove's Modified Dulbecco's medium (IMDM), MCDB131 medium, William's medium E, Fischer's medium, and the like.

Examples of the basal medium to be used when the medium of the present invention is prepared particularly for culturing stem cells (particularly pluripotent stem cell) include STEMPRO (registered trade mark) hESC SFM medium (Life Technologies), mTeSR1 medium (STEMCELL Technologies), TeSR2 medium (STEMCELL Technologies), TeSR-E8 medium (STEMCELL Technologies), Essential 8 medium (Life Technologies), HEScGRO (trade mark) Serum-Free medium for hES cells (Millipore), PluriSTEM (trade mark) Human ES/iPS medium (EMD Millipore), NutriStem (registered trade mark) hESC XF medium (Biological Industries Israel Beit-Haemek), NutriStem (trade mark) XF/FF Culture medium (Stemgent), AF NutriStem (registered trade mark) hESC XF medium (Biological Industries Israel Beit-Haemek), S-medium (DS pharma biomedical), StemFit (registered trade mark) AK03N medium (Ajinomoto Co., Inc.), hESF9 medium, hESF-FX medium, CDM medium, DEF-CS 500 Xeno-Free 3D Spheroid Culture medium (Cellartis), StemFlex medium (Thermo Fisher Scientific), and the like.

Also, in addition to the basal medium, components preferable for cellular proliferation can also be further added to the medium of the present invention. Examples of such component include sugars such as glucose, fructose, sucrose, maltose, and the like; amino acids such as asparagine, aspartic acid, glutamine, glutamic acid, and the like; proteins such as albumin, transferrin, and the like; peptides such as glycylglycylglycine, soybean peptide, and the like; serum; vitamins such as choline, vitamin A, vitamin Bs (thiamine, riboflavin, pyridoxine, cyanocobalamin, biotin, folic acid, pantothenic acid, nicotine amide etc.), vitamin C, vitamin E, and the like; fatty acids such as oleic acid, arachidonic acid, linoleic acid, and the like; lipids such as cholesterol and the like; inorganic salts such as sodium chloride, potassium chloride, calcium chloride, magnesium sulfate, sodium dihydrogen phosphate, and the like; trace elements such as zinc, copper, selenium, and the like; buffering agents such as N,N-bis(2-hydroxyethyl)-2-aminoethanesulfonic acid (BES), N-[tris(hydroxymethyl)methyl]glycine (Tricine), and the like; antibiotics such as amphotericin B, kanamycin, gentamicin, streptomycin, penicillin, and the like; cell adhesion factors and extracellular matrix components such as Type I collagen, Type II collagen, fibronectin, laminin, poly-L-lysine, poly-D-lysine, and the like; cytokines and growth factors such as interleukin, fibroblast growth factor (FGF), hepatocyte growth factor (HGF), transforming growth factor (TGF)-α, transforming growth factor (TGF)-β, vascular endothelium growth factor (VEGF), activin A, and the like; hormones such as dexamethasone, hydrocortisone, estradiol, progesterone, glucagon, insulin, and the like; and the like. Appropriate components can be selected and used according to the type of the cells to be cultured.

The medium of the present invention is practiced by adding HEPES to a basal medium such that the final concentration is not less than 30 mM, and further adjusting the osmotic pressure to not more than 340 mOsm/kg. When the basal medium contains HEPES as a buffering agent, HEPES is added in an amount after subtracting such amount. The medium of the present invention is produced by adding HEPES to a basal medium such that the final concentration is not less than 30 mM. It is also preferable to set the final concentration to not less than 35 mM or not less than 40 mM.

From the aspect of osmotic pressure adjustment, HEPES is preferably added to the basal medium at a final concentration of not more than 190 mM. It is also preferably not more than 180 mM, not more than 170 mM, not more than 160 mM, not more than 150 mM, not more than 140 mM, not more than 130 mM, not more than 120 mM, not more than 110 mM, or not more than 100 mM.

The medium of the present invention is produced by adding HEPES at a final concentration of not less than 30 mM and not more than 190 mM. It is also preferably not less than 35 mM and not more than 180 mM, not less than 40 mM and not more than 170 mM, not less than 40 mM and not more than 160 mM, not less than 40 mM and not more than 150 mM, not less than 40 mM and not more than 140 mM, not less than 40 mM and not more than 130 mM, not less than 40 mM and not more than 120 mM, or not less than 40 mM and not more than 100 mM. Particularly preferably, the medium of the present invention is produced by adding HEPES to a basal medium at a final concentration of not less than 40 mM and not more than 160 mM.

In one preferred embodiment, D-glucose and five kinds of amino acids (tryptophan, serine, cysteine (or cystine), methionine, arginine) may be further added to the medium of the present invention. The amount of these components to be added can be appropriately set according to the purpose of the culture, various culture conditions (e.g., cell density, frequency of medium exchange), and the like, by referring to the corresponding description in "2. Cell culture method" below.

When the medium of the present invention is applied to stem cells (particularly pluripotent stem cell), in one preferred embodiment, bFGF and/or choline may be further added to the medium of the present invention.

The medium of the present invention is characterized in that it contains HEPES at a concentration not less than the concentration recommended so far and that the osmotic pressure is adjusted to an optimal range.

The osmotic pressure of the medium may be measured by a method known per se. The principle of osmotic pressure measurement includes boiling point elevation method, vapor pressure depression method, freezing point depression method, and the like. As a measurement device, indirect measurement by the freezing point depression method or vapor pressure depression method, and a diaphragm-type osmotic pressure measurement device are used. The medium of the present invention is not particularly limited as regards the measurement principle/measuring apparatus of the osmotic pressure as long as it contains HEPES at a concentration not less than the concentration recommended so far and the osmotic pressure is adjusted to an optimal range. At least the osmotic pressure measured by the measurement principle of the freezing point depression method, specifically, the osmotic pressure measured by the method described in the Examples is adjusted to a certain range.

The osmotic pressure of the medium of the present invention measured by the measurement principle of the freezing point depression method is not particularly limited as long as it is not more than 340 mOsm/kg. It is preferably not more than 330 mOsm/kg, more preferably not more than 320 mOsm/kg, particularly preferably not more than 310 mOsm/kg, further preferably not more than 300 mOsm/kg. The lower limit of the osmotic pressure is not particularly limited as long as the upper limit is as described above. It is generally 220 mOsm/kg, preferably 230 mOsm/kg, more preferably 240 mOsm/kg, particularly preferably 250 mOsm/kg. In one embodiment, the osmotic pressure of the medium of the present invention may be generally 220 to 340 mOsm/kg, preferably 230 to 330 mOsm/kg, more preferably 240 to 320 mOsm/kg, further preferably 250 to 310 mOsm/kg, particularly preferably 250 to 300 mOsm/kg. In another embodiment of the present invention, the osmotic pressure of the medium of the present invention is 238 to 340 mOsm/kg.

To adjust the osmotic pressure of the medium, for example, a method capable of reducing the amount of a medium component (other than HEPES) that contributes to an increase in the osmotic pressure can be mentioned. For example, the osmotic pressure may be reduced to a desired level by appropriately decreasing the amount of components such as sodium chloride, and the like in the basal medium. When sodium chloride is used as a medium component other than HEPES that contributes to an increase in osmotic pressure, it can be added to the medium at a final concentration within the range of 0 to 150 mM. Sodium chloride is used in the concentration range of preferably final concentration 2 to 145 mM, more preferably 3 to 140 mM, further preferably 3 to 130 mM. Alternatively, the osmotic pressure can also be easily adjusted by diluting the basal medium with an appropriate amount of water.

The cell type to which the medium of the present invention can be applied is not particularly limited. As such cell type, plant cell and animal cell can be mentioned, preferably animal cell. Examples of the animal cell include germ cells such as spermatozoon, ovum, and the like, somatic cells constituting the living body, stem cells (pluripotent stem cell, etc.), progenitor cells, cancer cells separated from the living body, cells (cell lines) that are separated from the living body, acquire immortalizing ability, and are stably maintained ex-vivo, cells that are separated from the living body and subjected to artificial gene modification, cells that are separated from the living body and subjected to artificial nuclear exchange, and the like. Examples of the somatic cell constituting the living body include, but are not limited to, fibroblast, bone marrow cell, B lymphocyte, T lymphocyte, neutrophil, erythrocyte, platelet, macrophage, monocyte, osteocyte, pericyte, dendritic cell, keratinocyte, adipocyte, mesenchymal cell, epithelial cell, epidermis cell, endothelial cell, vascular endothelial cell, hepatocyte, chondrocyte, cumulus cell, neuronal cells, glial cell, neuron, oligodendrocyte, micro glia, astrocyte, heart cell, esophageal cell, muscle cells (e.g., smooth myocyte or skeleton myocyte), pancreas beta cell, melanocyte, hematopoietic progenitor cell (e.g., CD34 positive cell derived from cord blood), mononuclear cell, and the like. The somatic cell includes, for example, cells taken from any tissue such as skin, kidney, spleen, adrenal gland, liver, lung, ovary, pancreas, uterus, stomach, colon, small intestine, large intestine, bladder, prostate, testis, thymus, muscle, connective tissue, bone, cartilage, vascular tissue, blood (including cord blood), bone marrow, heart, eye, brain, neural tissue, and the like.

Stem cell is a cell that has the ability to replicate itself and the ability to differentiate into other multi-lineage cells. Examples thereof include, but are not limited to, embryonal carcinoma cell, pluripotent stem cell, neural stem cell, hematopoietic stem cell, mesenchymal stem cell, hepatic stem cell, pancreatic stem cell, muscle stem cell, germ stem cell, intestinal stem cell, cancer stem cell, hair follicle stem cell, and the like.

The "pluripotent stem cell" means a cell having self-renewal potential and differentiation/proliferation potency and capable of differentiating into any tissue or cell constituting living organisms. Examples of the pluripotent stem cell include embryonic stem cell (ES cell), embryonic germ cell (EG cell), induced pluripotent stem cell (iPS cell), pluripotent stem cells induced and selected by stress and cell stimulation, and the like. Stem cells established by culturing early embryos produced by nuclear transfer of somatic cell nuclei are also preferred as pluripotent stem cells (Nature, 385, 810 (1997); Science, 280, 1256 (1998); Nature Biotechnology, 17, 456 (1999); Nature, 394, 369 (1998); Nature Genetics, 22, 127 (1999); Proc. Natl. Acad. Sci. USA, 96, 14984 (1999); Nature Genetics, 24, 109 (2000)). In the present invention, a cell preferred as the pluripotent stem cell is an iPS cell. Confirmation of iPS cell can be performed using an undifferentiation marker resulting from undifferentiated properties of iPS cell as an index. Examples of the undifferentiation marker include alkali phosphatase, Oct3/4, Sox2, Nanog, ERas, Esgl, and the like. Methods for detecting these undifferentiation markers include a method for detecting mRNA (use of primer and probe), an immunological detection method (use of antibody and label), and the like.

A cell line is a cell that has acquired infinite proliferation potency through artificial manipulation outside the body. Examples thereof include, but are not limited to, CHO (Chinese hamster ovary cell line), HCT116, Huh7, HEK293 (human fetal kidney cell), HeLa (human uterine cancer cell line), HepG2 (human liver cancer cell line), UT7/TPO (human leukemia cell line), MDCK, MDBK, BHK, C-33A, HT-29, AE-1, 3D9, Ns0/1, Jurkat, NIH3T3, PC12, S2, Sf9, Sf21, High Five (registered trade mark), Vero, and the like.

In one preferred embodiment, the cell is a stem cell, particularly a pluripotent stem cell, more preferably an iPS cell.

The medium of the present invention may be provided in a liquid state, or in a state of being concentrated more than the concentration at the time of use, or in a solid state such as freeze-dried powder and the like to be diluted with a solvent such as water and the like when in use, or dissolved or dispersed in a solvent such as water and the like before use.

### 2. Cell culture method and cell preparation method

The present invention also provides a method for culturing a cell, characterized by culturing in the medium (described above) of the present invention (hereinafter sometimes referred to as "the culture method of the present invention"). Using the medium of the present invention, cell proliferation efficiency can be improved. Therefore, the present invention also provides a method for preparing a cell, including a step of culturing and proliferating cells in the (aforementioned) medium of the present invention (hereinafter sometimes referred to as "the preparation method of the present invention").

In the present specification, the "adhesion culture" means to culture cells in the state wherein the cells are adhered to a culture vessel and the cells do not float in the culture medium even when the culture vessel is gently shaken during culture. It specifically means a method of culturing in an scaffold-dependent manner using a culture vessel with a surface treatment suitable for cell adhesion or a culture vessel coated with an extracellular matrix. Examples of the coating agent include Matrigel (BD Biosciences), Synthemax (Corning), gelatin, extracellular protein (e.g., collagen, laminin (e.g., laminin 111, 411 or 511), heparan sulfate proteoglycan, and entactin, etc.), a fragment of the extracellular protein, and a combination of these.

In the present specification, the "suspension culture" refers to a cell culture method performed in a state where cells do not adhere to the culture container. Suspension culture is one embodiment of the three-dimensional culture method. In the present invention, the suspension culture may or may not be accompanied by pressure from the outside or vibration on the liquid medium, or shaking or rotation operation in the liquid medium. While not particularly limited, suspension culture can be performed using one that is not artificially treated for the purpose of improving adhesiveness to cells (e.g., coating treatment by extracellular matrix and the like), or one that is artificially treated to suppress adhesiveness (e.g., coating treatment with polymer of polyhydroxyethyl methacrylic acid (poly-HEMA) or 2-methacryloyloxyethylphosphoryl choline) as a culture container.

Examples of the three-dimensional culture method include suspension culture, as well as embedded culture using soft agar, methylcellulose, or collagen gel as a matrix, culture using a holofiber-type bioreactor or a radial flow-type bioreactor, and the like.

In the present specification, the "high density culture" refers to a culture at a high cell density compared to the cell density (general density) expected in general cell culture. The criteria for high density only require contact between cells, and may vary depending on the culture method (adhesion culture/suspension culture, etc.), cell type, and the like. For example, in the case of suspension culture of iPS cells, it is exemplified by culture at a density of not less than 6×10⁵ cells/mL (preferably, not less than 2×10⁶ cells/mL).

The frequency of medium exchange in cell culture is generally determined in comprehensive consideration of various conditions such as cell density (general density/high density), culture method (adhesion culture/suspension culture), type of cell to be cultured, medium composition, culture conditions (temperature, gas concentration), amount of medium to be exchanged (total amount/partial amount), cost of medium, lifestyle of workers, and the like. It is generally once every two to three days, once a day, or multiple times (e.g., twice) a day. The medium exchange can also be performed at such frequency in the method of the present invention.

While it varies depending on the type of cell to be cultured, the cells are passaged when they reach a certain number or more, generally not less than about 80% confluent. The medium of the present invention can also be used or is preferably used in cell culture after passage.

On the other hand, taking note of one aspect of the present invention that the osmotic pressure of the medium is maintained within a range preferred for cell proliferation, the timing of the medium exchange can also be set to a time point when the osmotic pressure deviates from a certain level (for example, not more than 340 mOsm/kg, not more than 330 mOsm/kg, more preferably not more than 320 mOsm/kg, particularly preferably not more than 310 mOsm/kg, further preferably not more than 300 mOsm/kg; or 220 to 340 mOsm/kg, preferably 230 to 330 mOsm/kg, more preferably 240 to 320 mOsm/kg, further preferably 250 to 310 mOsm/kg, particularly preferably 250 to 300 mOsm/kg; or 238 to 340 mOsm/kg).

One embodiment to which the method of the present invention can be preferably applied includes high density culture using a bioreactor or the like. Specifically, when high density culture is performed using a bioreactor or the like, the pH of the medium may decrease due to the vital activity of a large number of cells. The culture method of the present invention is characterized in that the medium of the present invention containing HEPES at a high concentration and superior in the buffer potency is used. Thus, the pH does not decrease easily compared with conventional culture methods.

In the culture method of the present invention, the whole amount of the medium being used may be exchanged or a part (e.g., 10%, 20%, 30%, 40%, 50%, 60%, 70%, 80%, 90%, etc. based on the total amount of the medium in use) thereof may be exchanged at the time of exchange with the medium of the present invention. The amount of the medium to be exchanged is not particularly limited as long as the osmotic pressure after the medium exchange can be within a range suitable for cell culture (that is, not more than 340 mOsm/kg, not more than 330 mOsm/kg, more preferably not more than 320 mOsm/kg, particularly preferably not more than 310 mOsm/kg, further preferably not more than 300 mOsm/kg; or 220 to 340 mOsm/kg, preferably 230 to 330 mOsm/kg, more preferably 240 to 320 mOsm/kg, further preferably 250 to 310 mOsm/kg, particularly preferably 250 to 300 mOsm/kg; or 238 to 340 mOsm/kg) .

In one preferred embodiment, D-glucose and five kinds of amino acids (tryptophan, serine, cysteine (or cystine), methionine, arginine) may be further added to the medium of the present invention.

Glucose (or a salt thereof) can be added to the medium of the present invention such that the concentration converted to glucose concentration is generally 0.1 g/L/day to 900 g/L/day, preferably 1 g/L/day to 200 g/L/day, more preferably 1 g/L/day to 20 g/L/day.

In addition, five kinds of amino acids (tryptophan, serine, cysteine (cystine), methionine, and arginine) can be added to the medium of the present invention such that the concentration of tryptophan (concentration after conversion to tryptophan in a free form) is generally 0.1 mg/L/day to 11000 mg/L/day, preferably 1 mg/L/day to 1000 mg/L/day, more preferably 1 mg/L/day to 100 mg/L/day, the concentration of serine (concentration after conversion to serine in a free form) is generally 0.1 mg/L/day to 425000 mg/L/day, preferably 1 mg/L/day to 1000 mg/L/day, more preferably 1 mg/L/day to 100 mg/L/day, the concentration of cysteine or cystine (concentration after conversion to cysteine in a free form) is generally 0.1 mg/L/day to 280000 mg/L/day, preferably 1 mg/L/day to 1000 mg/L/day, more preferably 1 mg/L/day to 100 mg/L/day, the concentration of methionine (concentration after conversion to methionine in a free form) is generally 0.1 mg/L/day to 55000 mg/L/day, preferably 1 mg/L/day to 1000 mg/L/day, more preferably 1 mg/L/day to 100 mg/L/day, and the concentration of arginine (concentration after conversion to arginine in a free form) is generally 0.1 mg/L/day to 150000 mg/L/day, preferably 1 mg/L/day to 2000 mg/L/day, more preferably 1 mg/L/day to 200 mg/L/day.

When the culture method of the present invention is applied to stem cells, in one preferred embodiment, bFGF and/or choline may be further added to the medium of the present invention.

In the culture method of the present invention, culture conditions are not particularly limited, and a method known per se may be selected according to the cell type, cell density (general density/high density), culture method (adhesion culture/suspension culture, etc.) and the like. For example, the culture temperature may be generally 25°C to 39°C, preferably 33°C to 39°C. The carbon dioxide concentration may be generally 4% by volume to 10% by volume, preferably 4% by volume to 6% by volume. The oxygen concentration may be generally 1% by volume to 25% by volume, preferably 4% by volume to 20% by volume.

### 3. Cell-containing composition

The present invention also provides a cell-containing composition containing the medium of the present invention (mentioned above) and cells (hereinafter sometimes referred to as "the cell-containing composition of the present invention").

As used herein, the cell to be contained is a viable cell which is to be the target of culture in the medium of the present invention. As the cells, those exemplified in the above-mentioned "1. Medium for cell culture and production method thereof" can be mentioned. It is preferably an animal cell, more preferably a stem cell, particularly preferably a pluripotent stem cell (particularly iPS cell). The cell may be before or after culture in the medium of the present invention culture.

The present invention is explained in more detail in the following by referring to Examples, which do not limit the scope of the present invention in any manner.

### [Example]

In this Example, the osmotic pressure was measured using GONOTEC OSMOMAT (registered trade mark) 030 after calibration with MilliQ water, 100 and 500 mOsmol/kg Calibration Standard (GONOTEC). This measurement device is based on the measurement principle of the freezing point depression method.

### Example 1

### (Medium preparation)

### Medium with varying HEPES concentration without adjusting osmotic pressure (Table 1)

DMEM/F-12 (ThermoFisher SCIENTIFIC, 12500) (10 g), MilliQ water, and Essential 8^{™} Supplement (ThermoFisher SCIENTIFIC) (10 ml) were mixed. An aqueous NaHCO₃ solution (Sigma Aldrich) was added to a final concentration of 20.75 mM, and an aqueous HEPES solution (Sigma Aldrich) was added to a final concentration of 15 to 60 mM. The pH was adjusted to 7.4±0.15 using 1 M sodium hydroxide aqueous solution or 1 M hydrochloric acid. The pH was measured at room temperature using METTLER TOLEDO SevenExcellence.

**[Table 1]**

| medium name | HEPES final concentration (mM) | osmotic pressure (mOsm kg⁻¹) |
|---|---|---|
| A1 | 15 | 292 |
| A2 | 36 | 317 |
| A3 | 60 | 351 |

### Medium with varying HEPES concentration with adjusting osmotic pressure (Table 2)

An aqueous HEPES solution was added to StemFit (registered trade mark) AK03N (Ajinomoto Co., Inc.) at a final concentration of 12 to 153 mM. The pH was adjusted to 7.4±0.15 using 1 M sodium hydroxide aqueous solution or 1 M hydrochloric acid. The sodium chloride concentration was adjusted to set the osmotic pressure to any value shown in Table 2.

**[Table 2]**

| medium name | HEPES final concentration (mM) | osmotic pressure (mOsm/kg) |
|---|---|---|
| B1 | 12 | 293 |
| B2 | 43 | 296 |
| B3 | 73 | 294 |
| B4 | 153 | 310 |
| C1 | 43 | 238 |
| C2 | 43 | 249 |
| C3 | 43 | 258 |
| C4 | 43 | 268 |
| D1 | 43 | 267 |
| D2 | 43 | 319 |
| D3 | 43 | 337 |
| D4 | 43 | 373 |

### Test method

To rh-VTN-N (Gibco (registered trade mark)) or iMatrix-511(nippi)-coated culture container were added a test medium (A1 to A3, B1 to B4, C1 to C4, D1 to D4) (1.5 ml/well), and Y-27632 (Wako) 10 µM, and human iPS cells (iPS Academia Japan, 201B7 strain) were seeded at 2.0×10⁴ or 1.3×10⁴ cells/well. The cells were cultured under the conditions of 37°C, 5% CO₂. After 24 hr from the seeding, the medium was exchanged with a test medium free of Y-27632, after which the medium was exchanged once in 1 to 3 days. Every time, the culture supernatant was sampled immediately before medium exchange and the pH was measured within 10 min at room temperature. On day 6 (d6) or day 7 (d7), the cells in each well were detached using TrypLE^{™} select CTS^{™} (Gibco (registered trade mark)), and the cells in the suspension were counted by an automated cell viability analyzer Vi-CELL (BECKMAN COULTER).

### Form observation

Observation with a confocal microscope (OLYMPUS CKX41) showed no significant abnormalities in cell or colony morphology in both groups in which media (A1 - A3) with varying HEPES concentrations without osmotic adjustment were used, and media (B1 - B4, C1 - C4, D1 - D4) in which the HEPES concentration was changed by adjusting the osmotic pressure, by changing the sodium chloride concentration.

### Number of viable cells

The number of viable cells at the time point of d6 in culture using media A1 to A3 is shown in Fig. 1 (a). As the HEPES concentration increased, namely, along with an increase in the osmotic pressure, the number of viable cells tended to decrease. In medium (A3) added with 60 mM HEPES, the osmotic pressure increased to 351 mOsm/kg.

The number of viable cells at the time point of d7 in culture using media B1 to B4, C1 to C4, and D1 to D4 adjusted to various osmotic pressures are shown in Fig. 1 (b), (c) and (d) .

When prepared while maintaining a low osmotic pressure, higher numbers of viable cells were obtained in media added with 43 and 73 mM HEPES (B2, B3) than with 12 mM (B1). Even in the medium (B4) added with 153 mM HEPES, the osmotic pressure was suppressed to 310 mOsm/kg, and a sufficiently high number of viable cells was obtained. From the above, it was demonstrated that even if a high concentration of HEPES is added to a medium, the cytotoxicity is suppressed by adjusting the osmotic pressure, and the cell proliferation rate is improved due to the pH stabilizing effect.

In medium D4, which shows a high osmotic pressure of 373 mOsm/kg, the cell proliferation rate was lower than in other groups.

### pH change

In cell culture using media B1 to B4, the pH of the culture supernatant immediately before medium exchange was measured, and plotted against the culture period. The results are shown in Fig. 2. It was found that the pH value was stabilized with respect to the culture period as the HEPES concentration becomes higher. Generally, the production amount of lactic acid increases and the pH decreases as the number of cells increases. However, even in the B2 and B3 media, which had the highest number of viable cells, stable pH values were shown, indicating a high buffering action.

### Evaluation of undifferentiation rate

To an iMatrix-511 (nippi)-coated 6-well plate were added media B1 and B3 (1.5 mL/well), and Y-27632 (10 µM), and human iPS cells (201B7 strain) were seeded at 1.3×10⁴ cells/well. The cells were cultured under the conditions of 37°C, 5% CO₂, n=3. After 24 hr from the seeding, the medium was exchanged with a medium free of Y-27632, after which the medium was exchanged once in 1 to 3 days. On day 7, surface marker analysis was performed according to the following procedure. Using Accutase, the cells in each well were detached. The separated 2.0×10⁵ cells were washed with a buffer, 5-fold diluted PE Mouse Anti-Human CD30 (BD Pharmingen^{™}) or PE Mouse IgG1, κ Isotype Ctrl (ICFC) Antibody (Biolegend) was added by 20 µL,, and the mixture was stood for 20 min. After washing several times with the buffer, it was analyzed using Guava (registered trade mark) easyCyte^{™} flow cytometer (Luminex). From the obtained CD30 antigen positive rate (Fig. 3), it was shown that the cells cultured in the media B1 and B3 all maintained high undifferentiation rates.

### Evaluation of trigerm layers differentiation potential

The iPS cells (201B7 strain) cultured for 3 weeks using media B1 and B3 were further cultured for 2 weeks in an exchanged differentiation medium. The obtained cells were evaluated for changes in the undifferentiation marker and differentiation marker expression levels. Differentiation culture was performed according to the following procedure. As the differentiation medium, a mixture of general differentiation medium StemFit for Differentiation (Ajinomoto Co., Inc., AS401) and DMEM/F12 (1:1) (Thermo Fisher Scientific) at a volume ratio of 1:4 was used. The cells suspended in a differentiation medium containing Y-27632 (10 µM) were added at 2×10⁴ cells/well to a 96-well plate (SUMITOMO BAKELITE CO., LTD., PrimeSurface (registered trade mark) 96 Slit-well Plate) to form an embryoid body, and cultured for 2 weeks under the conditions of 37°C, 5% CO₂. Culture was performed at n=3. After two days from the seeding, the medium was exchanged with a differentiation medium free of Y-27632, after which 70% of the medium was exchanged once in 1 to 3 days. RNA was extracted and purified from the cells before differentiation culture and the embryoid body obtained on day 14 (d14) of differentiation culture, using Maxwell (registered trade mark) 16 LEV simplyRNA Purification Kits (Promega). Furthermore, reverse transcription was performed using PrimeScript (registered trade mark) RT Master Mix (Takara Bio). Using Nanog as an undifferentiation marker and SOX1, SNAI2, and SST primers for SYBR (registered trade mark) Green as trigerm layer differentiation markers, the expression level of each gene was evaluated by a real-time PCR thermal cycler. The relative expression levels of cells cultured in medium B1 based on the state of the cells before differentiation culture are shown in Fig. 4.

Fig. 4 shows that the cells cultured in medium B3 before differentiation culture express undifferentiation marker at the same level as the cells cultured in medium B1. In addition, it was confirmed that the undifferentiation marker tended to decrease and the trigerm layer differentiation markers tended to increase due to the differentiation culture in which the embryoid bodies were formed. From the above, it was suggested that the cells cultured in medium B3 for 3 weeks had the same undifferentiation degree and the same trigerm layer differentiation potential as medium B1.

### Comparison of pH buffer capacity (buffer potency)

50 mL of medium B1 or B3 was weighed and titrated with 0.1N hydrochloric acid (JUNSEI CHEMICAL) using an automatic titrator (Hiranuma Industry, COM-1600). Changes in the pH value with respect to the amount of hydrochloric acid added dropwise are shown in Fig. 5. In the pH range from 7.4 to 6.6, the pH decreased linearly with respect to the amount of hydrochloric acid added dropwise, and the slope thereof suggests that the pH buffer capacity of medium B3 was about 3.5 times that of medium B1.

### Example 2

### Suspension culture

By a method similar to that in Example 1, media B1' and B3' with varying HEPES concentration and adjusted osmotic pressure were produced.

**[Table 3]**

| medium name | HEPES final concentration (mM) | osmotic pressure (mOsm kg⁻¹) |
|---|---|---|
| B1' | 12 | 265 |
| B3' | 71 | 266 |

To a 30-mL bioreactor (Biott, BWV-S03A) were added medium B1' and B3' (30 mL), Y-27632 (10 µM), and human iPS cells (1210B2 strain) were seeded at 1.8×10⁷ cells/well. The cells were cultured with stirring under the conditions of 37°C, 5% CO₂, 120 rpm. The culture was performed at n=3. After 2 days and 3 days from the seeding, 70% of the medium was exchanged with a medium free of Y-27632. The pH of the culture supernatant was measured when the medium was exchanged on the second day (Fig. 6). Four days after seeding, the culture supernatant was removed by centrifugation, converted the cells to single cells by Accumax treatment and resuspended in the medium, and the number of viable cells was counted by Vi-CELL (Fig. 7). Surface marker analysis of the cells 4 days after the seeding was performed according to the following procedure. The separated cell sample (2.0×10⁵ cells) was washed with a buffer, 10-fold diluted PE Mouse Anti-Human CD30 (BD Pharmingen^{™}), PE Mouse IgG1, κ Isotype Ctrl (ICFC) Antibody (Biolegend), 5-fold diluted Alexa Fluor (registered trade mark) 647 Mouse anti-Human TRA-1-60 Antigen (BD Pharmingen^{™}), Alexa Fluor (registered trade mark) 647 Mouse IgG1 κ Isotype Control (BD Pharmingen^{™}) were each added by 20 pL, and the mixture was stood for 20 min. After washing several times with the buffer, they were analyzed using Attune NxT Flow Cytometer (Thermo Fisher Scientific). Separately, the cell sample (2×10⁵ cells) was washed with the buffer, subjected to a fixing treatment using BD Cytofix/Cytoperm^{™} Fixation/Permeablization Kit, 10-fold diluted Alexa Fluor (registered trade mark) 647 Mouse anti-SSEA-4 (BD Pharmingen^{™}) and Alexa Fluor (registered trade mark) 647 Mouse IgG1 κ Isotype Control (BD Pharmingen^{™}) were each added by 20 µL, and the mixture was stood for 20 min. After washing several times with the buffer, and the analysis was performed in the same manner. The positive rate of each marker is shown in Fig. 8. qPCR measurement was performed in the same manner as in the previous section by using Nanog and POU5F1 TaqMan probes as undifferentiation markers. The relative expression level of each target gene based on medium B1' is shown in Fig. 9.

From Fig. 7, it was shown that the number of viable cells about 1.9 times higher than B1 was obtained by using medium (B3') containing a high concentration of HEPES in 4 days of culture. From Fig. 6, it was shown that the pH of the supernatant of medium B3' was maintained higher than that of medium B1' as of day 2.

From Figs. 8 and 9, it was suggested that the high concentration of HEPES does not affect the undifferentiation rate because the surface marker positive rate of media B1' and B3' is of the same level, and the undifferentiated gene expression level is of the same level.

### Example 3

### Essential 8^{™} with enhanced buffer potency

A hypotonic Essential 8^{™} medium was prepared by using DMEM/F-12 (without NaCl, HEPES, NaHCO₃) (Thermo Fisher Scientific) instead of the Essential 8^{™} basal medium and adding the Essential 8^{™} Supplement. Sodium hydrogen carbonate was added to a final concentration of 21 mM. Furthermore, HEPES and sodium chloride were added to achieve the HEPES final concentration and osmotic pressure shown in Table 4, whereby media E1 to E4 were obtained.

**[Table 4]**

| medium name | HEPES final concentration (mM) | sodium chloride final concentration (mM) | osmotic pressure (mOsm/kg) |
|---|---|---|---|
| E1 | 15 | 94 | 293 |
| E2 | 30 | 84 | 292 |
| E3 | 75 | 48 | 291 |
| E4 | 140 | 1.6 | 292 |

To an iMatrix-511-coated 6-well plate were added media E1 to E4 (1.5 mL/well), and Y-27632 (10 µM), and human iPS cells (201B7 strain) were seeded at 1.3×10⁴ cells/well. The cells were cultured under the conditions of 37°C, 5% CO₂, n=3. After 24 hr from the seeding, the medium was exchanged with a medium free of Y-27632, after which the medium was exchanged once in 1 to 3 days. After 7 days, the cells in each well were detached using TrypLE^{™}select CTS^{™}, and passaged by seeding in a new 6-well plate.

From the increase rate for each passage (Fig. 10), the medium E3 containing 75 mM HEPES showed a higher increase rate than the medium E1 containing 15 mM HEPES, and an about 7.6-fold number of viable cells were obtained as a total of three passages. From the above, it was suggested that the cell proliferation rate is improved by increasing the HEPES concentration.

### [Industrial Applicability]

According to the present invention, it is possible to provide a medium with improved pH stability due to a superior buffering action afforded by a high HEPES concentration. Furthermore, the medium for cell culture, the cell culture method, and the like of the present invention can improve the cell proliferation rate. Therefore, a larger number of cells, particularly pluripotent stem cells such as iPS cells and the like, can be obtained, and a large amount of the cells can be supplied for use in research, medical care, and the like.

This application is based on a patent application No. 2020-054572 filed in Japan (filing date: March 25, 2020), the contents of which are incorporated in full herein by reference.

## Claims

1. A medium for cell culture, comprising 4-(2-hydroxyethyl)-1-piperazineethanesulfonic acid (HEPES) at a concentration of not less than 30 mM, and having an osmotic pressure of not more than 340 mOsm/kg.

2. The medium according to claim 1, wherein the osmotic pressure is not less than 220 mOsm/kg.

3. The medium according to claim 1 or 2, wherein the HEPES concentration is not more than 190 mM.

4. The medium according to any one of claims 1 to 3, wherein the HEPES concentration is not less than 30 mM and not more than 190 mM.

5. The medium according to any one of claims 1 to 4, wherein the cell is a pluripotent stem cell.

6. The medium according to claim 5, wherein the pluripotent stem cell is an iPS cell.

7. A method for producing a medium for cell culture, comprising adding HEPES to a basal medium at a final concentration of not less than 30 mM, and further adjusting the osmotic pressure to not more than 340 mOsm/kg.

8. The method according to claim 7, wherein the osmotic pressure is not less than 220 mOsm/kg.

9. The method according to claim 8, wherein the osmotic pressure is adjusted by adjusting a concentration of sodium chloride in the medium.

10. The method according to any one of claims 7 to 9, wherein the HEPES concentration is not more than 190 mM.

11. The method according to any one of claims 7 to 10,
wherein the HEPES concentration is not less than 30 mM and not more than 190 mM.

12. The method according to any one of claims 7 to 11,
wherein the cell is a pluripotent stem cell.

13. The method according to claim 12, wherein the pluripotent stem cell is an iPS cell.

14. A method for culturing a cell, comprising culturing in a medium according to any one of claims 1 to 4.

15. The method according to claim 14, wherein the cell is a pluripotent stem cell.

16. The method according to claim 15, wherein the pluripotent stem cell is an iPS cell.

17. A cell-containing composition, comprising a medium according to any one of claims 1 to 4 and a cell.

18. The composition according to claim 17, wherein the cell is a pluripotent stem cell.

19. The composition according to claim 18, wherein the pluripotent stem cell is an iPS cell.

20. A method for preparing a cell, comprising a step of culturing and proliferating the cell in a medium according to any one of claims 1 to 4.

21. The method according to claim 20, wherein the cell is a pluripotent stem cell.

22. The method according to claim 21, wherein the pluripotent stem cell is an iPS cell.
